# EUROPEAN PATENT SPECIFICATION

(11) **EP 1 409 365 B1**
(45) Date of publication and mention of the grant of the patent: **19.10.2005**
(21) Application number: 02767838.2
(22) Date of filing: 24.07.2002
(51) Int. Cl.: B65D 75/32, A61M 15/00

(54) **AUTOPERFORATION CARTRIDGE FOR DRY POWERED INHALATIONS**
SELBSTPERFORIERENDE KARTUSCHE FÜR TROCKEN-PULVER INHALATIONEN
CARTOUCHE AUTO-PERFORANTE POUR INHALATIONS DE POUDRES SECHES

(30) Priority: 24.07.2001 IT PG20010036
(43) Date of publication of application: 21.04.2004
(73) Proprietor: Cecchini, Marco, 06124 Perugia (IT); Deveglia, Roberta, 06129 Perugia (IT)
(72) Inventor: Cecchini, Marco, 06124 Perugia (IT); Deveglia, Roberta, 06129 Perugia (IT)
(74) Representative: Pizzoli, Pasquale Vincenzo
(86) International application number: PCT/IT2002/000486
(87) International publication number: WO 2003/011708

(56) References cited:
- EP-A- 0 129 985
- EP-A- 0 850 082
- WO-A-01/43529

## Description

### Technical Field

The present invention relates to the technical field of the manufacturing of cartridges for dry powdered inhalations for medicinal use.

### Background Art

Presently known inhalers are provided with perforating mechanisms such as spikes and rather complicated assemblies that require expensive machinery to manufacture the products.

The most efficient inhalers are provided with different items to be assembled (delivering body, spike, spring, grid, housing for the selected cartridge) with consequent costs for manufacturing and arranging the machinery for the assembly line that produces the finished product.

In order to save money and to simplify their use, some inhalers have been designed in an extremely simple and essential way, i.e. breathable bags, elongated breathable capsules.

In that case the extreme simplification may cause wrong manoeuvres, misunderstandings, confusion and accidental breakage.

When using an inhaler, effectiveness, strength, functionality and pressure resistance should be guaranteed and they cannot be obtained with means such as paper materials, bags and pipes.

EP 0 850 082 discloses a cartridge for dry powdered inhalations for medicinal use consisting of a protective concave shell provided with an opening closed by a tearable film, i.e. a press-through packaging or "blister", and containing a rigid and hollow insert. The shell and the film make the powder damp-proof and pollutant-proof, while the insert allows to transfer the pressure exerted on the shell directly to the film so that no pressure is exerted on the powder contained in the cartridge. In this way, the powder remains finely dispersed and completely suitable for inhalation by the user.

Such a cartridge is defined as "autoperforating" cartridge in that it does not require cartridge-opening means (e.g. spike) but only pressure-exerting means.

However, also this type of cartridge is not exempt from drawbacks due to the fact that the several embodiments disclosed in the above-mentioned European patent always include a separate insert that must be introduced in the blister cavity either prior to the powder load or thereafter, or even is pre-loaded with the powder, and is then completely expelled from the cartridge upon opening of the latter.

This results in at least three drawbacks both in manufacturing and use:
a) the necessity for the separate production and introduction of the insert, which results in more manufacturing steps and more complicated machinery;
b) the impossibility of determining a precise orientation in the opening, since the insert goes completely through the opening as it is a separate element; and
c) the presence of the insert in the powder drop area that can hinder the inhalation of the powder by the user.

Moreover, such a cartridge is still used in a rather complicated inhaler made up of several parts required to advance the multidose cartridge and place it in the correct position where a pushbutton presses the insert out of the blister.

### Summary of the invention

The present invention relates to an autoperforating cartridge that overcomes the above-mentioned drawbacks by means of a rigid frame, located inside the shell, that is an integral part of the rigid base structure of the cartridge itself. In other words, the frame is a shaped structure that is pivoted through a joint on the rigid base of the cartridge and is manufactured therewith.

Upon application of a pressure on the shell, the frame leans and takes position in the passage of the inhaler, yet is still connected to the structure of the cartridge itself. The frame can be made in the shape of an asymmetric structure, but can be also made as a cone-shaped or dome-shaped structure, anyway following the shape of the cartridge where it is to be located.

A first important advantage of the cartridge according to the present invention is the reduction in manufacturing costs resulting from the fact that the frame is produced together with the cartridge structure, whereby at least two manufacturing steps (insert production and insert introduction) are dispensed with, and the powder load is easier. Also, such a cartridge can be fitted on different inhalers, preferably disposable and monodose but also multidose, that can be much simpler since they do not require advancing and pressure-exerting means, since the latter can even simply be the user's finger. This results in a dramatic reduction in the number of pieces of the inhaler, which in turn means cost reduction for the machinery required for assembling the selected inhaler.

A second significant advantage stems from the possibility of selecting the position of the joint between the frame and the cartridge base, so that the protective film will be torn starting from an area opposite to the joint area and there will be the possibility of properly orienting the powder drop.

Still another great advantage results from the permanent connection between the insert and the cartridge base, whereby the frame leans into the inhaler but does not completely and uncontrollably drop down together with the powder so that it does not risk to be an obstacle to the inhalation of the powder by the user. On the contrary, it can be provided with a properly mesh-shaped distal portion to act as a grid for the disaggregation of the medicinal powder from the excipient, in case the inhaler is not provided with a suitable grid.

### Brief description of the drawings

Further advantages and characteristics of the cartridge according to the present invention will be clear to those skilled in the art from the following detailed description of some embodiments thereof, with reference to the annexed drawings wherein:
**Fig.1** is a perspective see-through view of the cartridge in its assembled state;
**Fig.2a** is a perspective view of a first embodiment of the plastic frame;
**Fig.2b** is a perspective view of a second embodiment of the plastic frame;
**Fig.3a** is a perspective view that shows a transparent embodiment of the powder-receiving shell with its relevant base portion;
**Fig.3b** is a view similar to the preceding one of a semi-opaque shell;
**Fig.3c** is a bottom view of the present cartridge showing an embodiment with a pre-established break line in the protective film;
**Fig.3d** is a view similar to the preceding one showing a reinforced area of the protective film;
**Fig.4a** is a perspective diagrammatic view of the present cartridge placed on a simple tubular inhaler; and
**Fig.4b** is a view similar to the preceding one showing the cartridge in the opened state.

### Detailed description of the preferred embodiments

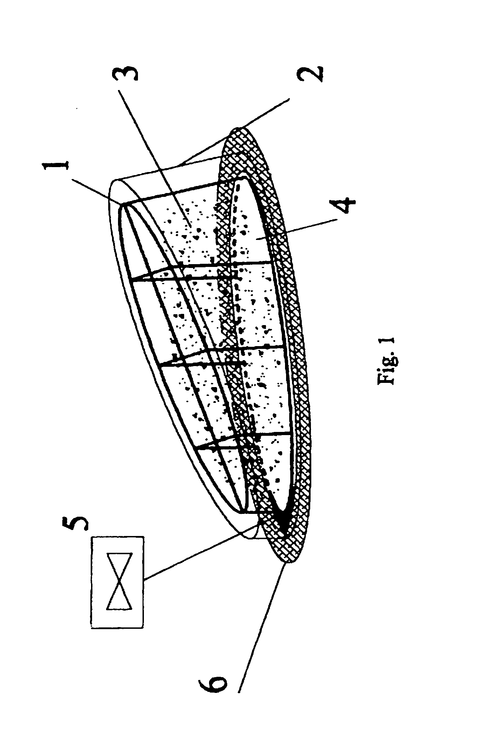

With reference to fig.1, there is seen that a cartridge according to the present invention includes a plastic frame 1, in this case asymmetrically shaped with a trapezoidal longitudinal section, placed inside an equally-shaped shell 2 that receives and protects the medicinal powder 3, the bottom of the cartridge being closed by a protective film 4.

The novel aspect of the present invention consists in a flexible joint 5 that permanently connects frame 1 to the rigid cartridge base 6. Such a connection can be achieved through different types of joint, in particular a "butterfly" snap-joint (shown in the boxed enlargement of joint 5) that through a raised bridge with lateral recesses provides an amplification of the push on the frame and prevents the reverse movement of the latter.

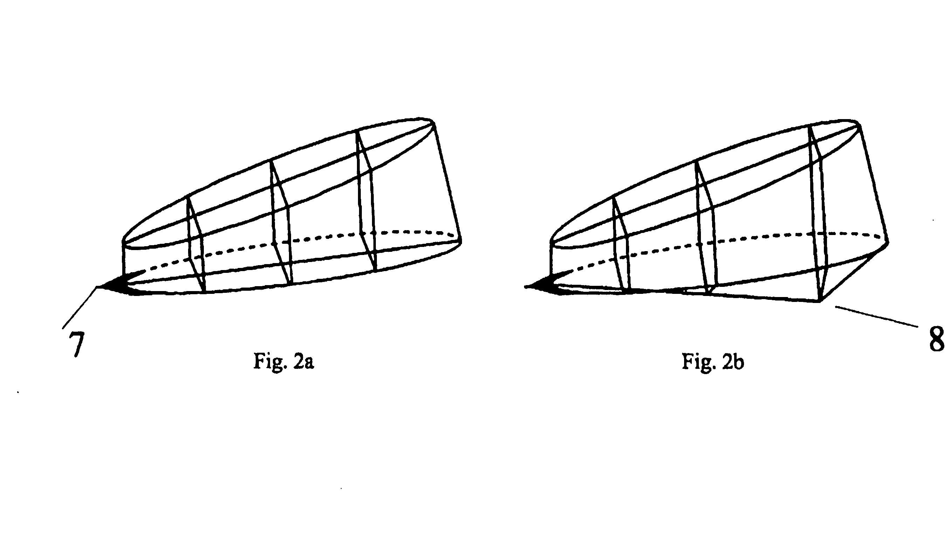

This type of joint is clearly useful when frame 1 is intended to remain in the leaning position upon squashing of the cartridge, for example if frame 1 is mesh-shaped to act as a grid, but other types of joints may be provided that do not have the non-return function. In this respect, fig.2a shows a joint 7consisting of a simple flexible strip.

The piercing of film 4 is simply achieved by the external pressure applied on frame 1 through shell 2, however frame 1 can also be suitably shaped to make the piercing operation easier. For example, as shown in fig.2b, it can be provided with a distal bottom angle 8 that easily starts a central line of tear.

The asymmetric shape of frame 1 illustrated in the figures, in which the distal part that in use will be adjacent to the inlet of the inhalation duct has a larger convexity than the proximal part where joint 5 is provided, is designed so that the piercing of film 4 occurs at the distal end of the cartridge. In this way, powder 3 tends to slide along the tearing film 4 and to drop closer to the inhalation duct rather than dropping backward as it could occur with prior art separate piercing inserts.

In order to let the user check that all the powder actually dropped down from the cartridge, shell 2 can be made from a transparent material 9 (fig.3a). However, in case the medicinal substance requires protection from light, shell 2 can be made from a semi-opaque material 10 (fig.3b) that provides the required protection while still allowing a visual check of the proper discharge of the powder.

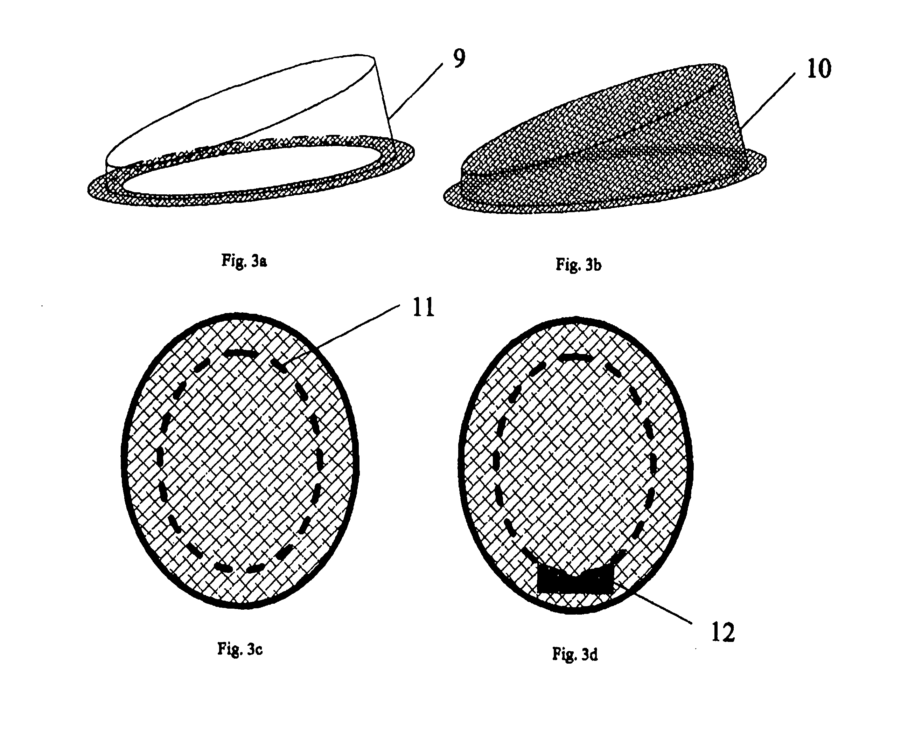

Fig.3c illustrates how another way to make the cartridge opening easier is to form in the bottom protective film 4 a dashed, as shown in the drawing, or continuous groove 11 that defines a preferential break line. In this way the pressure on the cartridge required to tear film 4 is lower, whereby even weak users such as ill, elderly or very young people can easily carry out the squashing required to drop the powder into the inhaler.

Although frame 1 can not be completely expelled from the cartridge thanks to the connection to base 6, nonetheless a reinforced area 12 can be provided along the above-mentioned groove 11 so as to assure the correct orientation in the film tear. Such a reinforcement also guarantees that the torn film is not completely detached from the cartridge, thus risking to drop down with the powder, which would be detrimental to the subsequent inhalation by the user.

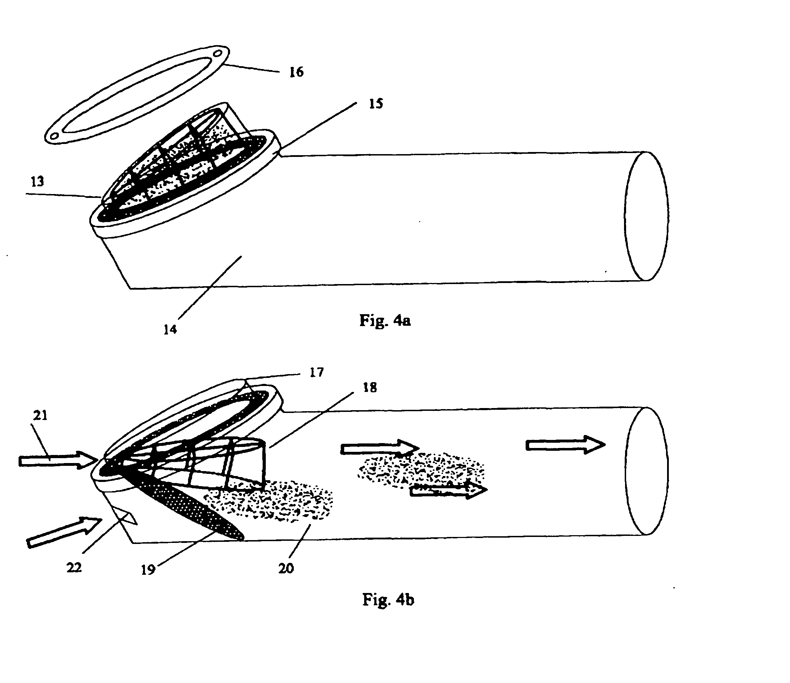

Finally, the application of a cartridge according to the present invention is illustrated by way of example in figs.4a and 4b. A cartridge 13 is placed in a simple and essential inhaler consisting of a tubular duct 14 provided with a suitable end lodging seat 15 formed in an oblique portion of the distal part of duct 14.

The cartridge can be fixed in different ways, such as by an adhesive agent arranged thereon, pressing, embedding, threading or even by placing a lock ring 16. Once the cartridge has been fixed in the inhaler, the user is simply required to press the cartridge so that the internal rigid frame tears open the protective film and allows the powder to drop down into the inhaler from where it can be inhaled by the user.

This operation is illustrated in fig.4b, where numeral 17 refers to the crushed shell, numeral 18 refers to the rigid frame 1 leaning inside the inhaler, numeral 19 refers to the torn protective film 4 and numeral 20 refers to the powdered medicament 3 now ready to be inhaled. It should be noted that both frame 18 and film 19 are still connected to the rigid basal part of the cartridge, whereby there is no risk of their inhalation by the user and they do not stand in the way of powder 20.

Said powder will be subjected to lateral and rear inhalation streams (arrows 21), that enter the inhaler through one or more air intakes 22 and carry the powder toward the proximal duct outlet where it enters the user's mouth. The air intakes can also be formed in the rigid cartridge base surrounding the central drug-holding part, so as to obtain other air streams that cooperate in transporting the powder.

The rigid structure of the cartridge, comprising frame 1 and base 6, will be made of a plastic, polypropylene, polycarbonate, nylon or metal material (or any other material suitable for the purpose). Said rigid structure is firstly lined in its convex rear part with a material selected for the blister to form shell 2, then it is filled with the medicinal powder and finally sealed with the protective film 4 on the base part.

As previously mentioned, the cage-like structure of frame 1 can be made with a narrow mesh, at least in the distal portion, when a further system of disaggregation of the powder particles in addition to the one usually provided with the inhaler itself is needed. In fact, the disaggregation of the medicinal substance from the excipient (usually lactose) is caused by the impact onto the filtering grid and by the strength of the inhalation streams.

It is clear that the above-described and illustrated embodiments of the cartridge according to the invention are just examples susceptible of various modifications. In particular, the exact shape and size can be freely changed according to specific needs, and the same applies to the materials used for the various elements that make up the cartridge (frame, shell, base, etc.).

## Claims

1. Cartridge for dry powdered inhalations (3) including a shaped rigid frame (1) placed inside an equally-shaped crushable shell (2) suitable to receive and protect said powder (3), the bottom of the cartridge being closed by a tearable protective film (4) applied to a rigid cartridge base (6), **characterized in that** said rigid frame (1) is pivoted on said base (6) through a flexible joint (5; 7) that permanently connects the frame (1) to the base (6).

2. Cartridge according to claim 1, **characterized in that** the flexible joint is a "butterfly" snap-joint (5) that provides an amplification of the push on the frame (1) and prevents the reverse movement of the latter.

3. Cartridge according to claim 1, **characterized in that** the flexible joint is a simple flexible strip (7) that does not provide a non-return function.

4. Cartridge according to one of the preceding claims, **characterized in that** the rigid frame (1) is provided with a distal bottom angle (8) suitable to start a central line of tear in the protective film (4).

5. Cartridge according to one of the preceding claims, **characterized in that** the shell (2) is made from a transparent (9) or semi-opaque (10) material suitable to allow the user to check that all the powder (3) actually dropped down from the cartridge.

6. Cartridge according to one of the preceding claims, **characterized in that** the bottom protective film (4) is provided with a dashed or continuous groove (11) that defines a preferential break line.

7. Cartridge according to claim 6, **characterized in that** a reinforced area (12) of the film (4) is provided along the groove (11).

8. Cartridge according to one of the preceding claims, **characterized in that** the frame (1) has an asymmetric shape in which the distal part that in use will be adjacent to the inlet of the inhalation duct of an inhaler has a larger convexity than the proximal part where the joint (5) is provided.

9. Cartridge according to one of the preceding claims, **characterized in that** the rigid base (6) is provided with restraining means suitable to block the cartridge in a corresponding seat of an inhaler, such as an adhesive agent, a thread, a snap-fitting or the like.

10. Cartridge according to one of the preceding claims, **characterized in that** the rigid base (6) is provided with one or more passages suitable to act as air intakes for inhalation streams.

11. Cartridge according to one of the preceding claims, **characterized in that** the structure of the frame (1) is made with a narrow mesh, at least in the distal portion, suitable to act as a grid for the disaggregation of the medicinal substance from the excipient.

12. Monodose inhalation system **characterized in that** it consists of an autoperforating cartridge (13) according to one of the preceding claims and an inhaler with a suitable cartridge-receiving seat (15).

13. Monodose inhalation system according to claim 12, **characterized in that** the cartridge-receiving seat (15) is formed in an oblique portion of the distal part of the inhaler duct (14).

14. Monodose inhalation system according to claim 12 or 13, **characterized in that** it includes a lock ring (16) suitable to lock the cartridge (13) in its seat (15).

## Patentansprüche

1. Kartusche für Trocken-Pulver Inhalationen (3), enthaltend einen geformten starren Rahmen (1), der in ein zusammendrückbares Gehäuse (2) von gleicher Form gesetzt ist, welches geeignet ist, das Pulver (3) aufzunehmen und zu schützen, wobei der Boden der Kartusche durch eine abziehbare Schutzfolie (4) verschlossen ist, die auf eine starre Grundfläche (6) der Kartusche aufgebracht ist, **dadurch gekennzeichnet, dass** der starre Rahmen (1) an der Grundfläche (6) durch ein elastisches Gelenk (5; 7) schwenkbar verbunden ist, welches den Rahmen (1) dauerhaft mit der Grundfläche (6) verbindet.

2. Kartusche nach Anspruch 1, **dadurch gekennzeichnet, dass** das elastische Gelenk ein "schmetterlingsförmiges" Schnappgelenk (5) ist, welches eine Verstärkung des Drucks auf den Rahmen (1) bietet und die Umkehrbewegung des letzteren verhindert.

3. Kartusche nach Anspruch 1, **dadurch gekennzeichnet, dass** das elastische Gelenk ein einfacher flexibler Streifen (7) ist, der keine Funktion zur Verhinderung der Umkehrbewegung bietet.

4. Kartusche nach einem der vorhergehenden Ansprüche, **dadurch gekennzeichnet, dass** der starre Rahmen (1) mit einer am entfernten Ende angeordneten unteren Ecke (8) versehen ist, die geeignet ist, den Anfang einer zentralen Risslinie in der Schutzfolie (4) zu bilden.

5. Kartusche nach einem der vorhergehenden Ansprüche, **dadurch gekennzeichnet, dass** das Gehäuse (2) aus einem transparenten (9) oder semiopaken (10) Material besteht, das geeignet ist, dem Anwender die Möglichkeit der Überprüfung zu geben, dass alles Pulver (3) tatsächlich aus der Kartusche nach unten gefallen ist.

6. Kartusche nach einem der vorhergehenden Ansprüche, **dadurch gekennzeichnet, dass** die untere Schutzfolie (4) mit einer gestrichelten oder durchgehenden Rille (11) versehen ist, welche eine bevorzugte Bruchlinie definiert.

7. Kartusche nach Anspruch 6, **dadurch gekennzeichnet, dass** ein verstärkter Bereich (12) der Folie (4) entlang der Rille (11) vorgesehen ist.

8. Kartusche nach einem der vorhergehenden Ansprüche, **dadurch gekennzeichnet, dass** der Rahmen (1) eine asymmetrische Form besitzt, bei welcher der entfernte Teil, der beim Gebrauch an die Einlassöffnung des Inhalationsrohrs eines Inhalators angrenzt, eine größere Konvexität aufweist als der nahe gelegene Teil, an dem das Gelenk (5) angebracht ist.

9. Kartusche nach einem der vorhergehenden Ansprüche, **dadurch gekennzeichnet, dass** die starre Grundfläche (6) mit Haltevorrichtungen versehen ist, dazu geeignet, die Kartusche in einem entsprechenden Sitz eines Inhalators festzuhalten, beispielsweise ein Klebstoff, ein Gewinde, ein Schnappverschluss oder dergleichen.

10. Kartusche nach einem der vorhergehenden Ansprüche, **dadurch gekennzeichnet, dass** die starre Grundfläche (6) mit einem oder mehreren Durchlässen versehen ist, die geeignet sind, als Lufteinlässe für Inhalationsströme zu dienen.

11. Kartusche nach einem der vorhergehenden Ansprüche, **dadurch gekennzeichnet, dass** die Struktur des Rahmens (1) zumindest im entfernten Teil aus einem engmaschigen Netz besteht, welches geeignet ist, als Gitter für die Aufschließung der medizinischen Substanz aus dem Trägerstoff zu wirken.

12. Einmaldosis-Inhalationssystem, **dadurch gekennzeichnet, dass** es aus einer selbstperforierenden Kartusche (13) nach einem der vorhergehenden Ansprüche und aus einem Inhalator mit einem Sitz (15) zur Aufnahme der Kartusche besteht.

13. Einmaldosis-Inhalationssystem nach Anspruch 12, **dadurch gekennzeichnet, dass** der Sitz (15) zur Aufnahme der Kartusche in einem schräg stehenden Teil des entfernten Teils des Inhalatorrohrs (14) angebracht ist.

14. Einmaldosis-Inhalationssystem nach Anspruch 12 oder 13, **dadurch gekennzeichnet, dass** es einen Verschlussring (16) enthält, der dazu geeignet, ist, die Kartusche (13) in ihrem Sitz (15) festzuhalten.

## Revendications

1. Cartouche pour des inhalations sous forme de poudre sèche (3) comprenant un cadre rigide profilé (1) placé à l'intérieur d'une coque cassable (2) également profilée apte à recevoir et protéger ladite poudre (3), le fond de la cartouche étant fermé par un film protecteur déchirable (4) appliqué à une base de cartouche rigide (6) **caractérisée en ce que** ledit cadre rigide (1) pivote sur ladite base (6) par l'intermédiaire d'une articulation flexible (5 ; 7) qui connecte de manière permanente le cadre (1) à la base (6).

2. Cartouche selon la revendication 1, **caractérisée en ce que** l'articulation flexible est une articulation à encliquetage « papillon » (5) qui fournit une amplification de la poussée sur le cadre (1) et empêche le mouvement inverse de ce dernier.

3. Cartouche selon la revendication 1, **caractérisée en ce que** l'articulation flexible est une simple bande flexible (7) qui ne fournit pas une fonction anti-retour.

4. Cartouche selon l'une quelconque des revendications précédentes, **caractérisée en ce que** le cadre rigide (1) est prévu avec un angle inférieur distal (8) apte à former le point de départ d'une ligne de déchirure centrale dans le film protecteur (4).

5. Cartouche selon l'une quelconque des revendications précédentes, **caractérisée en ce que** la coque (2) est réalisée à partir d'un matériau transparent (9) ou semi opaque (10) apte à permettre à l'utilisateur de vérifier que l'ensemble de la poudre (3) a réellement été libéré de la cartouche.

6. Cartouche selon l'une quelconque des revendications précédentes, **caractérisée en ce que** le film protecteur inférieur (4) est prévu avec une rainure interrompue ou continue (11) qui définit une ligne de rupture préférentielle.

7. Cartouche selon la revendication 6, **caractérisée en ce qu'**une surface renforcée (12) du film (4) est prévue le long de la rainure (11).

8. Cartouche selon l'une quelconque des revendications précédentes, **caractérisée en ce que** le cadre (1) a une forme asymétrique dans laquelle la partie distale, qui en utilisation sera adjacente à l'entrée du conduit d'inhalation d'un inhalateur, a une convexité plus grande que la partie proximale où l'articulation (5) est prévue.

9. Cartouche selon l'une quelconque des revendications précédentes, **caractérisée en ce que** la base rigide (6) est prévue avec des moyens de maintien aptes à bloquer la cartouche dans un siège d'un inhalateur correspondant, tel qu'un agent adhésif, un filetage, un encliquetage ou similaire.

10. Cartouche selon l'une quelconque des revendications précédentes, **caractérisée en ce que** la base rigide (6) est prévue avec un ou plusieurs passages aptes à agir en tant qu'entrées d'air pour des courants d'inhalation.

11. Cartouche selon l'une quelconque des revendications précédentes, **caractérisée en ce que** la structure du cadre (1) est réalisée avec une maille étroite, au moins dans la partie distale, appropriée à agir en tant que grille pour la désagrégation de la substance médicinale par rapport à l'excipient.

12. Système d'inhalation monodose **caractérisé en ce qu'**il consiste en une cartouche autoperforante (13), selon l'une quelconque des revendications précédentes, et en un inhalateur avec un siège de réception de cartouche approprié (15).

13. Système d'inhalation monodose selon la revendication 12, **caractérisé en ce que** le siège de réception de cartouche (15) est formé dans une partie oblique de la partie distale du conduit d'inhalation (14).

14. Système d'inhalation monodose selon la revendication 12 ou 13, **caractérisé en ce qu'**il comprend une bague de verrouillage (16) apte à verrouiller la cartouche (13) dans son siège (15).
